(19) Europäisches Patentamt
European Patent Office
Office européen des brevets

(11) EP 4 778 592 A2

(12) EUROPEAN PATENT APPLICATION

(43) Date of publication:
**22.07.2026 Bulletin 2026/30**

(21) Application number: **26178770.9**

(22) Date of filing: **20.10.2020**

(51) International Patent Classification (IPC):
***A61P 37/00*** *(2006.01)*

(52) Cooperative Patent Classification (CPC):
**C07D 471/04**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.10.2019 US 201962927354 P**

(62) Document number(s) of the earlier application(s) in accordance with Art. 76 EPC:
**20882313.8 / 4 051 270**

(71) Applicant: **Chou, Yu-Cheng**
**Hsinchu City (TW)**

(72) Inventor: **Chou, Yu-Cheng**
**Hsinchu City (TW)**

(74) Representative: **Gill, Siân Victoria**
**Venner Shipley LLP**
**TIDE Bankside**
**8 Emerson Street**
**London SE1 9DU (GB)**

Remarks:
This application was filed on 14.05.2026 as a divisional application to the application mentioned under INID code 62.

# (54) 4-AMINO-IMIDAZOQUINOLINE COMPOUNDS AND USE THEREOF

(57) 4-Amino-imidazoquinoline compounds and use thereof are disclosed. The compounds of the invention are Toll-like receptor 7 (TLR7) and TLR8 dual agonists, which exhibit activities in inducing IL-12 and IP-10 expression without over-inducing IL-6. The TLR 7/8 dual agonists are potentially useful medications as immune response modifiers. Use of a compound or salt thereof according to the invention in the manufacture of a medicament for treating a disease or a condition wherein activation of TLR7 and/or TLR8 provides a benefit in a subject in need thereof is disclosed. A compound or a pharmaceutical composition for use in treating a viral infection, cancer, and/or an allergic disease, or for use in activating immune responses that are effective against a viral infection, a tumor, and/or an allergic disease in a subject in need thereof is also disclosed.

Fig. 1A

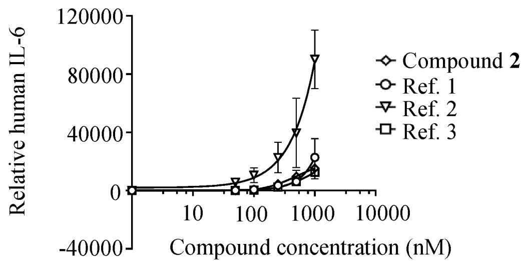


Fig. 1B

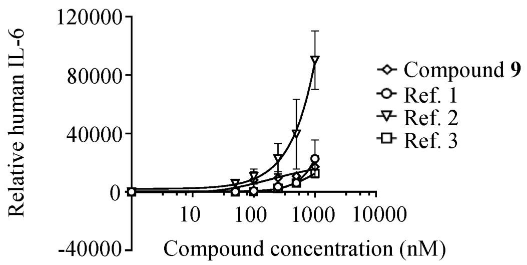


Fig. 1C

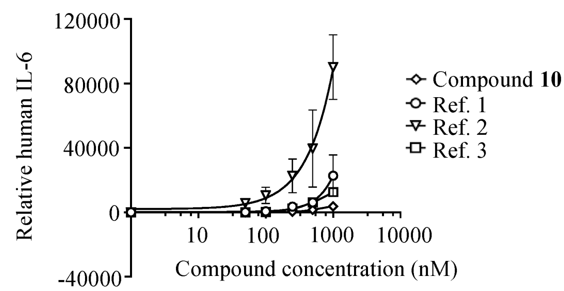


EP 4 778 592 A2

## Description

### FIELD OF THE INVENTION

The present invention relates generally to Toll-like receptor (TLR) agonists, and more specifically to TLR7 and TLR8 dual agonists.

### BACKGROUND OF THE INVENTION

Toll-like receptors (TLRs) are pathogen recognition receptors which play a critical role in activating both innate and adaptive immunity. Several TLRs have been identified in humans and mice. Some TLRs are found located on the cell surfaces (e.g., TLR 1, 2, 4, 5 and 6), and some are found located in the endosomal compartments (e.g., TLR 3, 7, 8 and 9). TLRs are expressed on different immune cells, especially monocytes, dendritic cells (DCs) and macrophages. The activation of TLRs results in cytokine secretion (e.g., IFN-$\alpha$, TNF-$\alpha$ and IL-12) and increased phagocytosis by macrophages and cytolytic activity by natural killer (NK) cells. TLRs activation also results in enhanced antigen presentation, thus activating an adaptive immune response, including antigen-specific $CD8^+$ cytotoxic T lymphocytes.

Small molecule TLR 7 and/or TLR 8 agonists have been identified in US Patent Nos. 8728486, 9334268, 9884866 and US Patent publication No. 20190062329. There is still a need for developing TLR7 and/or TLR8 agonists, especially TLR 7/8 agonists with improved potency and/or cytokine profile.

### SUMMARY OF THE INVENTION

In one aspect, the invention relates to a compound of formula (I)

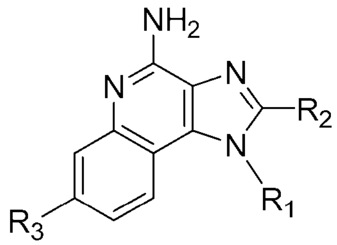

(I)

or a pharmaceutically acceptable salt thereof,
wherein
$R_1$ is hydroxy, hydroxy-$C_{1-6}$-alkyl, $C_{1-6}$-alkoxy, or $C_{1-6}$-alkoxy-$C_{1-6}$-alkyl;
$R_2$ is $C_{1-6}$-alkyl, $C_{1-6}$-haloalkyl, $C_{1-6}$-haloalkyl-O-$C_{1-6}$-alkyl, $C_{1-6}$-haloalkyl-S-$C_{1-6}$-alkyl, or hydroxy-$C_{1-6}$-alkyl; and
$R_3$ is hydrogen, hydroxy, halogen, $C_{1-6}$-alkyl, hydroxy-$C_{1-6}$-alkyl, $C_{1-6}$-haloalkyl, $C_{1-6}$-alkoxy, or $C_{1-6}$-alkoxy-$C_{1-6}$-alkyl;
wherein the $C_{1-6}$-alkyl, hydroxy-$C_{1-6}$-alkyl, $C_{1-6}$-alkoxy, $C_{1-6}$-alkoxy-$C_{1-6}$-alkyl, $C_{1-6}$-haloalkyl and $C_{1-6}$-haloalkyl-S-$C_{1-6}$-alkyl are each independently unsubstituted or substituted with at least one substituent; wherein the substituent is each independently selected from the group consisting of - $NH_2$, aryl, $C_{1-6}$-alkyl and $C_{1-6}$-alkyl-aryl;
with the proviso that,
when $R_1$ is 2-hydroxy-2-methylpropyl, $R_2$ is not methyl, ethyl, butyl or hydroxymethyl; and when $R_1$ is dimethylhydroxymethyl, $R_2$ is not isobutyl.

In another aspect, the invention relates to a compound of formula (II)

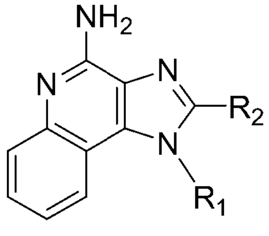

(II)

or a pharmaceutically acceptable salt thereof, wherein $R_1$ and $R_2$ are as defined above.

In one embodiment of the invention, $R_1$ is hydroxy-$C_{1-6}$-alkyl, or $C_{1-6}$-alkoxy-$C_{1-6}$-alkyl.

In another embodiment, $R_1$ is hydroxymethyl, 2-hydroxyethyl, 3-hydroxypropyl, 4-hydroxybutyl, or 2-hydroxy-2-methylpropyl.

Further in another embodiment, $R_1$ is methoxymethyl, 2-methoxyethyl, ethoxymethyl, or 2-ethoxyethyl.

In another embodiment, $R_2$ is straight-chain or branched $C_{1-6}$-alkyl.

In another embodiment, $R_2$ is methyl, ethyl, n-propyl, isopropyl, n-butyl, isobutyl, tert-butyl, n-pentyl, isopentyl, 1-methylbutyl, tert-pentyl, neopentyl, n-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, or 3,3-dimethyl-2-butyl.

In another embodiment, $R_2$ is sec-butyl, tert-butyl, or 3,3-dimethyl-1-butyl.

In another embodiment, $R_2$ is $C_{1-6}$-monohaloalkyl, $C_{1-6}$-dihaloalkyl or $C_{1-6}$-trihaloalkyl.

In another embodiment, $R_2$ is trifluoromethyl, 2,2,2-trifluoroethyl, 3,3,3-trfluoropropyl, 4,4,4-fluorobutyl, 5,5,5-trifluoropentyl, 6,6,6-trifluorohexyl, 3,3,3-trifluoro-2-methylpropyl, 4,4,4-trifluoro-3-methylbutyl, or 5,5,5-trifluoro-4-methylpentyl.

In another embodiment, $R_2$ is $-CH_2-CH_2-O-CH_2-CF_3$, $-CH_2-CH_2-O-CH_2-CH_2-CF_3$, $-CH_2-O-CH_2-CF_3$, $-CH_2-O-CH_2-CH_2-CF_3$, or $-CH_2-O-CH_2-CH_2-CH_2-CF_3$. In another embodiment, $R_2$ is $C_{1-6}$-haloalkyl-O-$C_{2-6}$-alkyl.

In another embodiment, $R_2$ is $-CH_2-CH_2-S-CH_2-CF_3$, $-CH_2-CH_2-S-CH_2-CH_2-CF_3$, $-CH_2-S-CH_2-CF_3$, $-CH_2-S-CH_2-CH_2-CF_3$, or $-CH_2-S-CH_2-CH_2-CH_2-CF_3$.

In another embodiment, $R_2$ is 2-hydroxyethyl, 3-hydroxypropyl, or 4-hydroxybutyl.

In another embodiment, $R_3$ is $C_{1-6}$-haloalkyl. The $C_{1-6}$-haloalkyl may be a trifluoroalkyl group.

In another embodiment, $R_3$ is H, OH, F, Cl, Br, I, or trifluoromethyl.

In another embodiment, $R_1$ is 2-hydroxy-2-methylpropyl, and $R_2$ is n-propyl, isobutyl, n-pentyl, isopentyl, 4-methylpentyl, trifluoromethyl, 2,2,2-trifluoroethyl, 3,3,3-trfluoropropyl, 4,4,4-fluorobutyl, 5,5,5-trifluoropentyl, 6,6,6-trifluorohexyl, 3,3,3-trifluoro-2-methylpropyl, 4,4,4-trifluoro-3-methylbutyl, 5,5,5-trifluoro-4-methylpentyl, $-CH_2-S-CH_2-CF_3$, $-CH_2-S-CH_2-CH_2-CF_3$, $-CH_2-S-CH_2-CH_2-CH_2-CF_3$, 2-hydroxyethyl, 3-hydroxypropyl, or 4-hydroxybutyl.

In another embodiment, $R_1$ is 2-hydroxy-2-methylpropyl, and $R_2$ is n-propyl, isobutyl, n-pentyl, 4,4,4-fluorobutyl, 5,5,5-trifluoropentyl or 3,3,3-trifluoro-2-methylpropyl.

In another embodiment, $R_1$ is 2-hydroxy-2-methylpropyl, $R_2$ is n-propyl, isobutyl, n-pentyl, 4,4,4-fluorobutyl, 5,5,5-trifluoropentyl, or 3,3,3-trifluoro-2-methylpropyl, and $R_3$ is H.

In another embodiment of the invention, a compound of formula (I) is selected from the compounds as listed in Table 2 of the present application.

In another embodiment, a compound of formula (I) according to the invention is selected from the group consisting of:

1-(4-amino-2-propyl-1*H*-imidazo[4,5-c]quinolin-1-yl)-2-methylpropan-2-ol (compound **1);**

1-(4-amino-2-pentyl-1*H*-imidazo[4,5-c]quinolin-1-yl)-2-methylpropan-2-ol (compound **2);**

1-(4-amino-2-isobutyl-1*H*-imidazo[4,5-c]quinolin-1-yl)-2-methylpropan-2-ol (compound **3);**

1-(4-amino-2-(4,4,4-trifluorobutyl)-1*H*-imidazo[4,5-c]quinolin-1-yl)-2-methylpropan-2-ol (compound **9);**

1-(4-amino-2-(5,5,5-trifluoropentyl)-1*H*-imidazo[4,5-c]quinolin-1-yl)-2-methylpropan-2-ol (compound **10);** and

1-(4-amino-2-(3,3,3-trifluoro-2-methylpropyl)-1*H*-imidazo[4,5-c]quinolin-1-yl)-2-methylpropan-2-ol (compound **12).**

A further aspect of the present invention is a process for the manufacture of compounds of formula (I).

In another aspect, the invention relates to a pharmaceutical composition comprising a compound of formula (I) or a pharmaceutically acceptable salt thereof as defined above, and a pharmaceutically acceptable carrier and/or an adjuvant.

In another aspect, the invention relates to use of a compound or a pharmaceutically acceptable salt thereof according to the invention in the manufacture of a medicament for treating a viral infection, cancer, an allergic disease or a liver disease in a subject in need thereof.

The invention also relates to a method of treating a viral infection, cancer, an allergic disease or a liver disease, said method comprising administering a therapeutically effective amount of a compound of formula (I) or a pharmaceutically acceptable salt thereof according to the invention to a subject in need thereof.

The invention also relates to a compound of formula (I) or a pharmaceutically acceptable salt thereof according to the invention for use in treating a viral infection, cancer, an allergic disease or a liver disease in a subject in need thereof.

In another embodiment, the viral infection is hepatitis C virus (HCV), hepatitis B virus (HBV), hepatitis D virus (HDV), human papillomavirus (HPV), or herpes simplex virus 1 (HSV-1), herpes simplex virus 2 (HSV-2) related infection.
In one embodiment, the cancer is selected from the group consisting of esophageal, stomach, colon, rectal, pancreatic, lung, breast, cervix uteri, corpus uteri, ovary, bladder, head and neck, endometrial, osteosarcoma, prostate, and neuroblastoma.
In another embodiment, the cancer is HER2 positive cancer, or PD-1 positive cancer.
In another embodiment, the allergic disease is allergic rhinitis (AR) or asthma.
In another embodiment, the liver disease is alcoholic fatty liver disease or nonalcoholic fatty liver disease (NAFLD). The NAFLD is selected from the group consisting of steatosis, nonalcoholic steatohepatitis (NASH), NASH-related fibrosis, and NASH-related cirrhosis.
Further in another aspect, the invention relates to use of a compound or a pharmaceutically acceptable salt thereof according to the invention in the manufacture of a medicament for treating a disease or a condition wherein activation of TLR7 and/or TLR8 provides a benefit in a subject in need thereof.
Yet in another aspect, the invention relates to use of a compound or a pharmaceutically acceptable salt thereof according to the invention in the manufacture of a medicament for activating immune responses that are effective against a viral infection, a tumor, an allergic disease, or a liver disease in a subject in need thereof.
These and other aspects will become apparent from the following description of the preferred embodiment taken in conjunction with the following drawings, although variations and modifications therein may be affected without departing from the spirit and scope of the novel concepts of the disclosure.
The accompanying drawings illustrate one or more embodiments of the invention and, together with the written description, serve to explain the principles of the invention. Wherever possible, the same reference numbers are used throughout the drawings to refer to the same or like elements of an embodiment.

## BRIEF DESCRIPTION OF THE DRAWINGS

Figs. 1A-C depict the relative IL-6 induction from human PBMCs stimulated with various concentrations of test compounds (compounds **2, 9** and **10)** and reference compounds **(Ref. 1-3).** Compound **Ref. 1** is resiquimod (a TLR7/8 dual agonist). Compound **Ref. 2** is 1-(4-amino-2-butyl-1*H*-imidazo[4,5-c]quinolin-1-yl)-2-methylpropan-2-ol (a TLR7/8 dual agonist). Compound **Ref. 3** is VTX2337 (a TLR8 agonist).

Figs. 2A-C depict the relative IL-12p70 induction from human PBMCs stimulated with various concentrations of test compounds and reference compounds. Test compounds and reference compounds are the same as Figs. 1A-C.

Figs. 3A-C depict the relative IP-10 induction from human PBMCs stimulated with various concentrations of test compounds and reference compounds. Test compounds and reference compounds are the same as Figs. 1A-C.

## DETAILED DESCRIPTION OF THE INVENTION

### DEFINITIONS

Unless otherwise defined, all technical and scientific terms used herein have the same meaning as commonly understood by one of ordinary skill in the art to which this invention belongs. Furthermore, the following definitions are set forth to illustrate and define the meaning and scope of the various terms used to describe the invention.
The nomenclature used in this application is based on IUPAC systematic nomenclature, unless indicated otherwise.
As used herein, the article "a" or "an" refers to one or to more than one (i.e. to at least one) of the grammatical object of the article. By way of example, "an element" means one element or more than one element. Furthermore, use of the term "including" as well as other forms, such as "include", "includes," and "included," is not limiting.
The term "hydroxy" or "hydroxyl" means the group -OH.
The term "alkyl", alone or in combination with other groups, refers to a branched or straight-chain monovalent saturated aliphatic hydrocarbon radical of one to twenty carbon atoms, particularly one to sixteen carbon atoms, more particularly one to ten carbon atoms. The term "alkyl" also embraces lower alkyl groups as described below.
The term "lower alkyl" or "$C_{1-6}$-alkyl", alone or in combination, signifies a straight-chain or branched-chain alkyl group with 1 to 6 carbon atoms, in particular a straight or branched-chain alkyl group with 1 to 5 carbon atoms and more particularly a straight or branched-chain alkyl group with 1 to 4 carbon atoms. Examples of straight-chain and branched $C_{1-6}$ alkyl groups are methyl, ethyl, n-propyl, isopropyl, n-butyl, isobutyl, tert-butyl, the isomeric pentyls (e.g., n-pentyl,

isopentyl, 1-methylbutyl, tert-pentyl, neopentyl), and the isomeric hexyls (e.g., n-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl or 3,3-dimethyl-2-butyl). Preferred are methyl, ethyl, n-propyl, isopropyl, n-butyl, isobutyl, sec-butyl, tert-butyl, n-pentyl, isopentyl, n-hexyl, 4-methyl-1-pentyl, and 3,3-dimethyl-1-butyl.

The term "hydroxy-$C_{1-6}$-alkyl" refers to lower alkyl groups as defined above wherein at least one of the hydrogen atoms of the lower alkyl group is replaced by a hydroxy group. Among the particular interesting hydroxy-$C_{1-6}$-alkyl groups are hydroxymethyl, 2-hydroxyethyl, 3-hydroxypropyl, 4-hydroxybutyl or 2-hydroxy-2-methylpropyl. In one embodiment, the hydroxy-$C_{1-6}$-alkyl group is 2-hydroxy-2-methylpropyl.

The term "$C_{1-6}$-alkoxy" refers to the group R'-O-, wherein R' is lower alkyl as defined above. Examples of $C_{1-6}$-alkoxy groups are methoxy, ethoxy, n-propoxy, isopropoxy, n-butoxy, isobutoxy, sec-butoxy and tert-butoxy. Preferred are methoxy, ethoxy, or n-propoxy.

The term "$C_{1-6}$-alkoxy-$C_{1-6}$-alkyl" refers to lower alkyl groups as defined above wherein at least one of the hydrogen atoms of the lower alkyl group is replaced by a $C_{1-6}$-alkoxy group as defined above. Among the $C_{1-6}$-alkoxy-$C_{1-6}$-alkyl groups of particular interest are methoxymethyl, 2-methoxyethyl, ethoxymethyl and 2-ethoxyethyl, with ethoxymethyl being of most particular interest.

The term "halo" or "halogen" by themselves or as part of another substituent, unless otherwise stated, refers to a fluorine (F), chlorine (Cl), bromine (Br), or iodine (I) atom. More particularly, halogen refers to fluorine or chlorine. Most particularly, halogen refers to fluorine. Additionally, terms such as "haloalkyl" are meant to include monohaloalkyl and polyhaloalkyl.

The term "$C_{1-6}$-haloalkyl" or "halogen-$C_{1-6}$-alkyl" refers to lower alkyl groups as defined above wherein at least one of the hydrogen atoms of the lower alkyl group is replaced by at least one halogen atom. Particularly, the $C_{1-6}$-haloalkyl group is $C_{1-6}$-monohaloalkyl, $C_{1-6}$-dihaloalkyl or $C_{1-6}$-trihaloalkyl. More particularly, the $C_{1-6}$-haloalkyl group is $C_{1-6}$-trihaloalkyl. Among the $C_{1-6}$-haloalkyl groups of particular interest are trifluoromethyl, 2,2,2-trifluoroethyl, 3,3,3-trfluoropropyl, 4,4,4-fluorobutyl, 5,5,5-trifluoropentyl, 6,6,6-trifluorohexyl, 3,3,3-trifluoro-2-methylpropyl, 4,4,4-trifluoro-3-methylbutyl and 5,5,5-trifluoro-4-methylpentyl, with 4,4,4-fluorobutyl, 5,5,5-trifluoropentyl or 3,3,3-trifluoro-2-methylpropyl being of more particular interest.

The term "$C_{1-6}$-haloalkyl-O-$C_{1-6}$-alkyl" or "$C_{1-6}$-haloalkoxy-$C_{1-6}$-alkyl" means lower alkyl groups as defined above, wherein one of the hydrogen atoms of the lower alkyl group is replaced by $C_{1-6}$-haloalkoxy group. For example, $C_{1-6}$-haloalkyl-O-$C_{1-6}$-alkyl group may be $-CH_2-CH_2-O-CH_2-CF_3$, - $CH_2-CH_2-O-CH_2-CH_2-CF_3$, $-CH_2-O-CH_2-CF_3$, $-CH_2-O-CH_2-CH_2-CF_3$, or $-CH_2-O-CH_2-CH_2-CH_2-CF_3$.

The term "$C_{1-6}$-haloalkyl-S-$C_{1-6}$-alkyl" or "$C_{1-6}$-haloalkylthio-$C_{1-6}$-alkyl" means lower alkyl groups as defined above, wherein one of the hydrogen atoms of the lower alkyl group is replaced by $C_{1-6}$-haloalkylthio group. For example, $C_{1-6}$-haloalkyl-S-$C_{1-6}$-alkyl group may be $-CH_2-CH_2-S-CH_2-CF_3$, $-CH_2-CH_2-S-CH_2-CH_2-CF_3$, $-CH_2-S-CH_2-CF_3$, $-CH_2-S-CH_2-CH_2-CF_3$ or $-CH_2-S-CH_2-CH_2-CH_2-CF_3$.

The terms"$C_{1-6}$" and "($C_1$-$C_6$)" are interchangeable.

By " $C_{1-6}$" or "($C_1$-$C_6$)", it means that all integer unit amounts within the range 1 to 6 are specifically disclosed as part of the invention. Thus, $C_1$, $C_2$, $C_3$, $C_4$, $C_5$, $C_6$; ($C_1$-$C_2$), ($C_1$-$C_3$), ($C_1$-$C_4$), ($C_1$-$C_5$), ($C_1$-$C_6$); ($C_2$-$C_3$), ($C_2$-$C_4$), ($C_2$-$C_5$), ($C_2$-$C_6$); ($C_3$-$C_4$), ($C_3$-$C_5$), ($C_3$-$C_6$); ($C_4$-$C_5$), ($C_4$-$C_6$); and ($C_5$-$C_6$) units amounts are included as embodiments of this invention.

The term "substituent" means an atom or a group of atoms replacing a hydrogen atom on the parent molecule.

The term "aryl" means a polyunsaturated, aromatic, hydrocarbon substituent, which can be a single ring or multiple rings (preferably from 1 to 3 rings) that are fused together (i.e., a fused ring aryl) or linked covalently. A fused ring aryl refers to multiple rings fused together wherein at least one of the fused rings is an aryl ring.

Compounds of formula (I) can form pharmaceutically acceptable salts. The term "pharmaceutically acceptable salts" refers to those salts which retain the biological effectiveness and properties of the free bases or free acids, which are not biologically or otherwise undesirable. Pharmaceutically acceptable salts include both acid and base addition salts. The salts are for example acid addition salts of compounds of formula (I) with physiologically compatible mineral acids, such as hydrochloric acid, hydrobromic acid, nitric acid, carbonic acid, sulfuric acid, sulfurous acid or phosphoric acid; or with organic acids, such as methanesulfonic acid, ethanesulfonic acid, p-toluenesulfonic acid, formic acid, acetic acid, propionic acid, glycolic acid, pyruvic acid, oxalic acid, lactic acid, trifluoroacetic acid, citric acid, fumaric acid, maleic acid, malonic acid, tartaric acid, benzoic acid, cinnamic acid, mandelic acid, embonic acid, succinic acid or salicylic acid. In addition, pharmaceutically acceptable salts may be prepared from addition of an inorganic base or an organic base to the free acid. Salts derived from an inorganic base include, but are not limited to, the sodium, potassium, lithium, ammonium, calcium, magnesium, zinc, copper, manganese and aluminium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, histidine, caffeine, procaine, hydrabamine, choline, betaine,

ethylendiamine, glucosamine, methylglucamine, theobromine, piperazine, N-ethylpiperidine, piperidine and polyamine resins. The compound of formula (I) can also be present in the form of zwitterions. Pharmaceutically acceptable salts of compounds of formula (I) of particular interest are the sodium salts or salts with tertiary amines.

The term "half maximal effective concentration ($EC_{50}$)" means the plasma concentration of a particular compound required for obtaining 50% of the maximum of a particular effect in vivo.

The term "therapeutically effective amount" means an amount of a compound of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, disease state being treated, the severity or the disease treated, the age and relative health of the subject, the route and form of administration, the judgment of the attending medical or veterinary practitioner, and other factors.

The term "treatment" or "treating" refers to the application or administration of a therapeutic agent, i.e. a compound provided herein, to a patient, or application or administration of a therapeutic agent to an isolated tissue or cell line from a patient (e.g., for diagnosis or ex vivo applications), who has a disease, a symptom of the disease or the potential to develop the disease, with the purpose to heal, alleviate, relieve, alter, remedy, ameliorate, improve or affect the disease, the symptoms of the disease, or the potential to develop the disease. Such treatments may be specifically tailored or modified, based on knowledge obtained from the field of pharmacogenomics.

The term "ammonia solution" may particularly denote a solution having a solvent to which ammonia is added, for example, ammonia may be solved in an aqueous solvent or an organic solvent. Ammonia is a compound of nitrogen and hydrogen with the formula $NH_3$. An ammonia solution may be a solution of $NH_3$ in water. A characteristic property of ammonia is its basicity so that it may form an alkaline solution with water, i.e. having a pH value above 7. In addition, an ammonia solution may a solution of $NH_3$ in methanol.

HER2 positive cancer cells have abnormally high levels of HER2 proteins. HER2 negative cancer cells do not have abnormal levels of HER2 proteins. PD-1 positive cancer cells have abnormally high levels of PD-1 proteins. PD-1 negative cancer cells do not have abnormal levels of PD-1 proteins.

The compounds of formula (I) and their pharmaceutically acceptable salts according to the invention may be used as a medicament, in a form of a pharmaceutical composition/formulation suitable for enteral, parenteral or topical route of administration. The medicament may be used for systemic (e.g., parenteral) or local (e.g., topical or intralesional injection) administration. The routes of administration of the medicament may be topically, parenterally, vaginally, intrauterine, intranasal, or by inhalation.

Certain tissues may be preferred targets for TLR agonist. In one embodiment, lymph nodes, spleen, bone marrow, blood, tumor locations and tissues exposed to virus are preferred sites of administration of the TLR agonists of the invention.

Techniques for preparing various dosage forms of parenteral, oral, transdermal, and pulmonary are all well-known in the skill of the art. Effective doses will vary, as recognized by those skilled in the art, depending on route of administration, excipient usage, and the possibility of co-usage with other therapeutic treatment.

The "Guidance for Industry and Reviewers Estimating the Safe Starting Dose in Clinical Trials for Therapeutics in Adult Healthy Volunteers" published by the U.S. Department of Health and Human Services Food and Drug Administration discloses "a human equivalent dose" may be obtained by calculations from the following formula:

$$\text{HED} = \text{animal dose in mg/kg} \times (\text{animal weight in kg/human weight in kg})^{0.33}.$$

## EXAMPLES

### Preparation of the compounds of the invention

### *General Synthesis Scheme*

Compounds of formula (I) may be made according to a general synthesis scheme shown below.

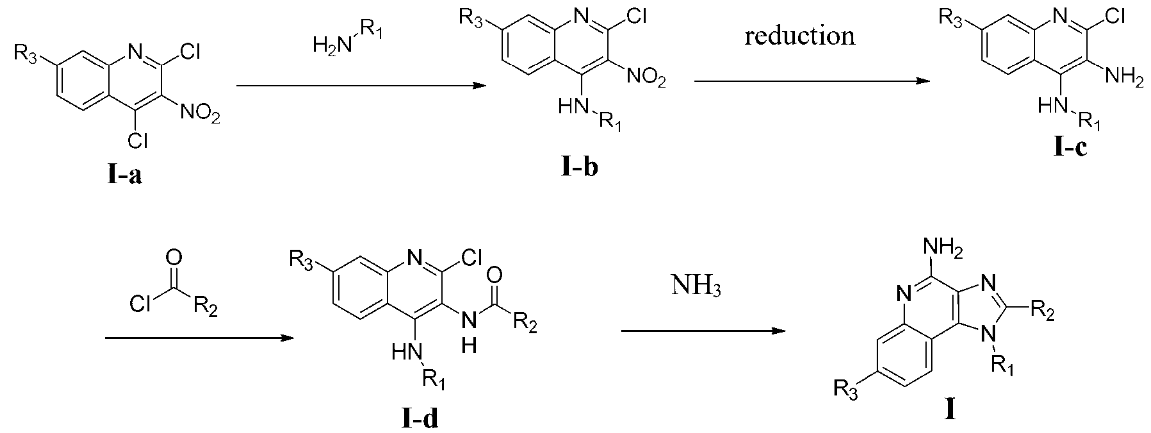


I-a I-b I-c I-d I

In general, a method of making a compound of formula (I) according to the invention comprises the steps of:

(a) reacting a compound of formula (I-a)

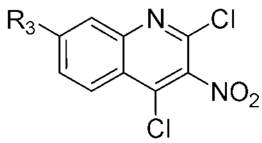

(I-a)

with $NH_2$-$R_1$ in the presence of a catalyst to obtain a compound of formula (I-b)

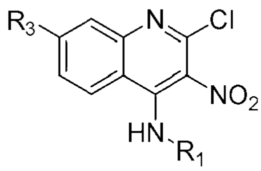

(I-b),

wherein $R_1$ and $R_3$ are as defined above;

(b) reducing the compound of formula (I-b) in the presence of a reducing agent to obtain a compound of formula (I-c);

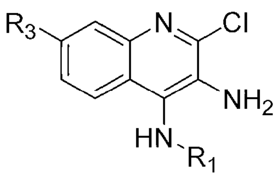

(I-c),

wherein $R_1$ and $R_3$ are as defined above;

(c) reacting the compound of formula (I-c) with C1-C(O)-$R_2$ to obtain a compound of formula (I-d)

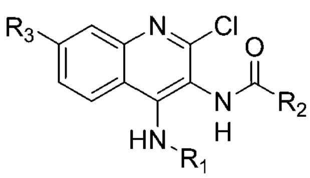

(I-d),

wherein $R_1$, $R_2$ and $R_3$ are as defined above; and

(d) reacting the compound of formula (I-d) with an ammonia solution to obtain the desired compound of formula (I)

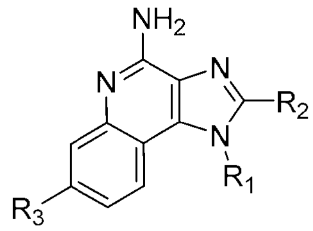

(I),

wherein $R_1$, $R_2$ and $R_3$ are as defined above.

In step (a), the compound of formula (I-b) may be obtained in the presence of a solution of triethylamine (TEA) in dichloromethane (DCM) at a temperature between 40°C to 60°C, preferably between 50°C to 60°C, or at 50°C for at least 1 hour, preferably for 1 to 4 hours, more preferably for 2 to 3 hours.

In step (b), the compound of formula (I-b) maybe reduced in the presence of Fe/$NH_4Cl$ at a temperature between 70°C to 100°C, preferably between 80°C to 90°C, or at 80°C for at least 1 hour, preferably for 1 to 4 hours, more preferably for 2 to 3 hours.

In step (c), the compound of formula (I-c) maybe dissolved in acetonitrile (ACN), and then reacts with C1-C(O)-$R_2$ at a temperature between 15°C to 35°C, or at a room temperature for at least 1 hour, preferably for 1 to 5 hours, more preferably for 2 to 4 hours..

In step (d), the desired compound of formula (I) may be obtained in the presence of a solution of $NH_3$ in methanol at a temperature between 120°C to 180°C, preferably between 130°C to 160°C, or at150°C for at least 24 hours, preferably for 30 to 50 hours, more preferably for 35 to 40 hours.

Compounds of Formula (I) according to the invention may be prepared using the above general scheme. Table 2 lists compounds of the invention. Synthesis of representative compounds are described in more detail below for illustration purpose.

***Synthesis of intermediate compound A***

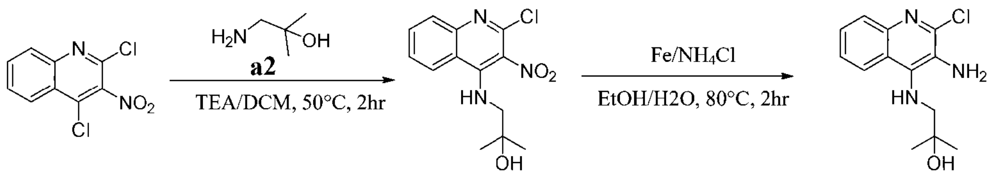


a1 a3 A

Scheme A

Compound **a1** was dissolved in dichloromethane (DCM) containing triethylamine (TEA) and was then selectively displaced by primary amine compound **a2** at 50°C for 2 hours to give nitroquinoline compound **a3.** Then, compound **a3** was mixed with EtOH/$H_2O$ and reduced by reacting with Fe/$NH_4Cl$ at 80°C for 2 hours to yield intermediate compound A (Scheme A). The resulting intermediate compound **A** was used in the following examples.

***Analysis of test compounds***

All reactions were monitored for completion by thin layer chromatography (TLC) using Merck 60 $F_{254}$ silica gel glass backed plates (20 × 20 cm). Visualization of the resulting chromatograms was detected visually under UV irradiation (254 nm).

To analyze test compounds, $^1H$ NMR spectra were recorded on Varian Mercury-400 spectrometers and the chemical shifts were recorded in parts per million (ppm, $\delta$). Multiplicities are recorded as s (singlet), br s (broad singlet), d (doublet), t (triplet), q (quartet), quin (quintet), sxt (sextet), and m (multiplet). Coupling constants (J) are expressed in hertz. For liquid chromatography-mass spectrometry (LC-MS), electrospray mass spectra (ESMS) were recorded as m/z values using Waters mass spectrometer. For high-performance liquid chromatography (HPLC), purity of the final compound was determined with Waters ACQUITY Arc system using C18 column (Waters XSelect HSS T3 column, 5 μm, 4.6 mm x 250

mm) operating at 40 °C. Elution was carried out using water containing 0.1% trifluoroacetic acid and methanol as mobile phases. The flow-rate of the mobile phase was 1 mL/min. Peaks were detected at 210-400 nm.

***Synthesis of Compound No. 1***

1-(4-amino-2-propyl-1*H*-imidazo[4,5-c]quinolin-1-yl)-2-methylpropan-2-ol (compound **1)** was synthesized according to scheme 1.

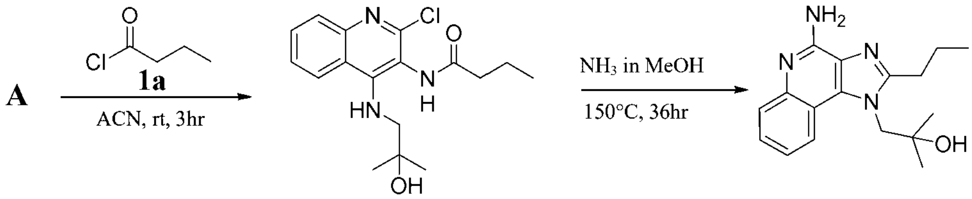


**1b** **1**

Scheme 1

Intermediate compound **A** was dissolved in acetonitrile (ACN) and reacted with butyryl chloride (compound No. **1a**) at room temperature for 3 hours to yield corresponding amine compound No. **1b**. Compound No. **1b** was reacted with ammonia solution (7N in methanol) at 150 °C for 36 hours to result in formation of an imidazole ring and displacement of the 4-chloro substituent to yield compound No. **1.** The crude product was purified by column chromatography to give the title compound as a beige solid. $^1$H NMR (400 MHz, DMSO-d6) δ 8.26 (d, *J* = 8.0 Hz, 1 H), 7.57 (d, *J* = 8.0 Hz, 1 H), 7.36 (t, *J* = 8.0 Hz, 1 H), 7.18 (t, *J* = 8.0 Hz, 1 H), 6.40 (s, 2 H), 4.78 (s, 1 H), 4.58 (br s, 2 H), 3.00 (t, *J* = 7.5 Hz, 2 H), 1.82 (sxt, *J* = 7.4 Hz, 2 H), 1.17 (s, 6 H), 1.00 (t, *J* = 7.4 Hz, 3 H). LCMS (ESI) *m/z* 299.3 $[M+H]^+$. HPLC Purity: 99.65%.

***Synthesis of Compound No. 2***

1-(4-amino-2-pentyl-1*H*-imidazo[4,5-c]quinolin-1-yl)-2-methylpropan-2-ol (compound 2) was synthesized according to scheme 2.

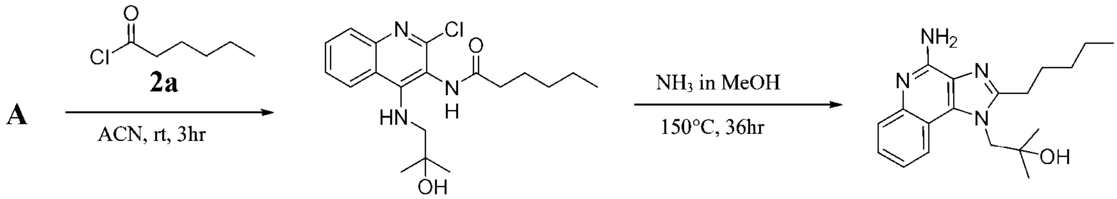


**2b** **2**

Scheme 2

Intermediate compound **A** was dissolved in acetonitrile (ACN) and then reacted with hexanoyl chloride (compound No. **2a)** at room temperature for 3 hours to yield corresponding amine compound No. **2b.** Compound No. **2b** was reacted with ammonia solution (7N in methanol) at 150 °C for 36 hours to result in formation of an imidazole ring and displacement of 4-chloro substituent to yield compound No. **2.** The crude product was purified by column chromatography to give the title compound as a beige powder. $^1$H NMR (400 MHz, DMSO-d6) δ 8.25 (d, *J* = 7.8 Hz, 1 H), 7.57 (d, *J* = 7.8 Hz, 1 H), 7.36 (t, *J* = 7.8 Hz, 1 H), 7.18 (t, *J* = 7.8 Hz, 1 H), 6.40 (s, 2 H), 4.78 (s, 2 H), 4.54 (br s, 2 H), 3.01 (t, *J* = 7.8 Hz, 2 H), 1.80 (quin, *J* = 7.2 Hz, 2 H), 1.49-1.28 (m, 4 H), 1.17 (s., 6 H), 0.89 (t, *J* = 7.2 Hz, 3 H). LCMS (ESI) *m/z* 327.4 $[M+H]^+$. HPLC Purity: 99.42%.

***Synthesis of Compound No. 9***

1-(4-amino-2-(4,4,4-trifluorobutyl)-1*H*-imidazo[4,5-c]quinolin-1-yl)-2-methylpropan-2-ol (compound No. 9) was synthesized according to scheme 4.

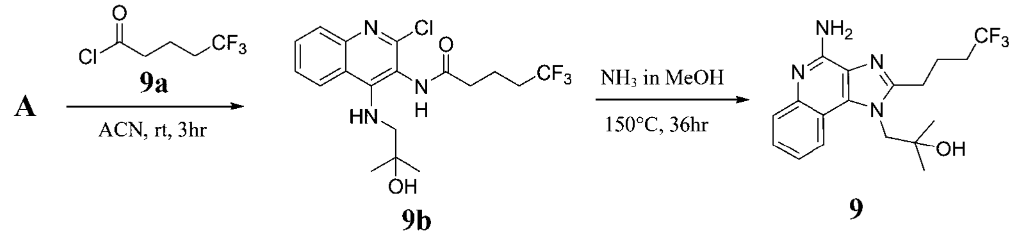


Scheme 4

Intermediate compound **A** was dissolved in acetonitrile (ACN) and reacted with 5,5,5-trifluoropentanoyl chloride (compound No. **9a)** at room temperature for 3 hours to yield corresponding amine compound No. **9b.** Compound No. **9b** was reacted with ammonia solution (7N in methanol) at 150 °C for 36 hours to result in formation of an imidazole ring and displacement of the 4-chloro substituent to yield compound No. **9.** The crude product was purified by column chromatography to give the title compound as a white powder. $^1$H NMR (400 MHz, DMSO-*d6*) δ 8.26 (d, *J* = 7.8 Hz, 1 H), 7.58 (d, *J* = 7.8 Hz, 1 H), 7.37 (t, *J* = 7.8 Hz, 1 H), 7.19 (t, *J* = 7.8 Hz, 1 H), 6.44 (s, 2 H), 4.80 (s, 1 H), 4.55 (br s, 2 H), 3.12 (t, *J* = 7.5 Hz, 2 H), 2.48-2.34 (m, 2 H), 2.06 (quin, *J* = 7.5 Hz, 2 H), 1.17 (s., 6 H). LCMS (ESI) *m/z* 367.4 $[M+H]^+$. HPLC Purity: 96.95%.

***Synthesis of Compound No. 10***

1-(4-amino-2-(5,5,5-trifluoropentyl)-1*H*-imidazo[4,5-c]quinolin-1-yl)-2-methylpropan-2-ol (compound No. **10)** was synthesized according to scheme 5.

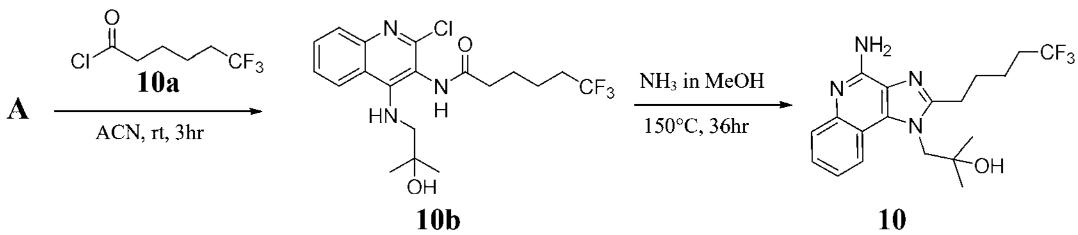


Scheme 5

Intermediate compound **A** was dissolved in acetonitrile (ACN) and reacted with 6,6,6-trifluorohexanoyl chloride (compound No. **10a)** at a room temperature for 3 hours to yield corresponding amine compound No. **10b.** Compound No. **10b** was reacted with ammonia solution (7N in methanol) at 150 °C for 36 hours to result in formation of an imidazole ring and displacement of the 4-chloro substituent to yield Compound No. **10.** The crude product was purified by column chromatography to give the title compound as a white powder. $^1$H NMR (600 MHz, DMSO-d6) δ 8.26 (d, *J* = 8.4 Hz, 1H), 7.57 (dd, *J* = 8.4, 1.2 Hz, 1H), 7.36 (ddd, *J* = 8.4, 7.0, 1.3 Hz, 1H), 7.20 (ddd, *J* = 8.4, 7.0, 1.3 Hz, 1H), 6.37 (s, 2H), 4.48 (s, 1H), 4.55 (br s, 2H), 3.06 (t, *J* = 7.8 Hz, 2H), 2.37-2.29 (m, 2H), 1.90 (quintet, *J* = 7.8 Hz, 2H), 1.64 (quintet, *J* = 7.8 Hz, 2H), 1.17 (br s, 6H). LCMS (ESI) *m/z* 381.4 $[M+H]^+$. HPLC purity: 99.34%.

Using similar synthesis schemes described above, other compounds listed in Table 2 could be readily generated. For example, Compound No. **3** was prepared using a procedure similar to the synthesis of compound 1 but using 3-methylbutanoyl chloride instead of butyryl chloride. Compound No. **22** was prepared using a procedure similar to the synthesis of Compound No. 9, but using 3-amino-2-chloro-4-((2-hydroxy-2-methylpropyl)amino)quinolin-7-ol instead of Intermediate compound **A.**

***In vitro TLR7 and TLR8 agonistic activity analysis***

To analyze the hTLR7 agonistic activity, HEK-Blue hTLR7 cells transfected with secreted embryonic alkaline

phosphatase (SEAP) reporter were seeded at $3\times10^4$ cell/well. The cells were treated with various concentrations of test compounds (Compound Nos. **1, 2, 9, 10).** Two reference compounds were used for comparisons: **Ref. 1** (resiquimod, a TLR7/8 dual agonist) and **Ref. 2** (1-(4-amino-2-butyl-1*H*-imidazo[4,5-c]quinolin-1-yl)-2-methylpropan-2-ol. **Ref. 2** is a more potent TLR7/8 dual agonist than resiquimod. See Ganapathi L. *et al.* (2015), PLOS ONE, 10(8): e0134640). The SEAP reporter is designed specifically for measuring secreted placental AP (alkaline phosphatase) in conditioned cell culture medium from transfected cells. The HEK-Blue hTLR7 cells served to measure the bioactivity of TLR7 through SEAP upon NF-κB activation following TLR7 stimulation.

To analyze the hTLR8 agonistic activity, HEK-Blue hTLR8 cells transfected with SEAP reporter were seeded at $3\times10^4$ cell/well. The cells were treated with various concentrations of test compounds (Compound Nos. **1, 2, 9, 10).** Two reference compounds **Ref. 1** and **Ref. 2** were used for comparisons. The HEK-Blue hTLR8 cells served to measure the bioactivity of TLR8 through the SEAP upon NF-κB activation following TLR8 stimulation.

In the SEAP reporter gene assay as described above, water was used as a negative control. After about 16 hours of incubation at 37 °C in 5% $CO_2$, the SEAP was determined by using a spectrophotometer at a wavelength of 635 nm. The dose-response curves were fit with four parameter logistic curves (SigmaPlot, version 9.0). The $EC_{50}$-values of the test compounds and reference compounds were calculated. Table 1 shows the results of the analysis of TLR7 and TLR8 agonistic activities.

Table 1

| Compound No. | TLR7 $EC_{50}$ (nM) | TLR8 $EC_{50}$ (nM) | Ratio of TLR8 $EC_{50}$ and TLR7 $EC_{50}$ |
|---|---|---|---|
| **1** | 236.9 ± 7.4 | 5299.1 ± 14.4 | 22.36 |
| **2** | 67.1 ± 8.3 | 267.1 ± 13.5 | 3.98 |
| **9** | 128.8 ± 10.9 | 965.4 ± 14.7 | 7.49 |
| **10** | 542.8 ± 29.4 | 4745.6 ± 20.5 | 8.74 |
| **Ref. 1** | 161.9 ± 28.6 | 1698.2 ± 44.2 | 10.48 |
| **Ref. 2** | 24.0 ± 3.2 | 689.8 ± 27.5 | 28.74 |

Table 1 shows that all the test compounds exhibited TLR7 and TLR8 dual agonistic activities. Among those, Compound **No. 2** was the most potent TLR7/8 dual agonist due to its low $EC_{50}$ of TLR7 and TLR8 agonistic activity. In addition, the ratios of TLR8 $EC_{50}$ and TLR7 $EC_{50}$ of the Compound Nos. **2, 9, 10** were lower than the two reference compounds, which indicates that these compounds could bring advantages of activating TLR7 and TLR8 at a very closed potency ratio and precisely modulating the activation of TLR7 and TLR8.

***Ex vivo cytokines profile analysis***

Three test compounds (Compound Nos. **2, 9, 10)** were used to evaluate the effect on cytokine induction. Three reference compounds were used for comparisons: **Ref. 1**(resiquimod, a TLR7/8 dual agonist), **Ref. 2** (1-(4-amino-2-butyl-1*H*-imidazo[4,5-c]quinolin-1-yl)-2-methylpropan-2-ol, a TLR7/8 dual agonist) and **Ref. 3** (Motolimod or VTX2337, a TLR8 agonist).

Cryopreserved human PBMCs from 4 normal adult volunteers were purchased from EPBMC® (IMMUNOSPOT®). The PBMCs were thawed at 37°C for 10 minutes and centrifuged at 300×g at room temperature for 5 minutes. The pelleted PBMCs were transferred to RPMI-1640 medium containing 10% FBS, 1% PSA and incubated at 37°C with 5% CO2.

The test compounds and reference compounds were respectively dissolved in DMSO and subsequently 25-fold diluted with PBS to make stock solutions at a concentration of 0.1 mM. The preincubated PBMCs were harvested and resuspended in a fresh RPMI-1640 medium containing 10% FBS and 1% PSA. Approximately $1\times10^5$ PBMCs were added to each well of 96-well plates and stimulated with 0, 20 or 1000 nM of test compounds for 24 h at 37°C in a 5% $CO_2$ humidified incubator. The culture supernatant was stocked at -20°C for analysis of TLR7- and TLR8-mediated cytokine induction. PROCARTAPLEX™ Immunoassay (Invitrogen) was performed to quantify human IL-6, IL-12p70, IP-10 in the culture supernatant according to the manufacturer's instructions. Briefly, the culture supernatant samples were thawed on ice and transferred to a microtiter plate. Magnetic bead-coupled antibodies specific to the target cytokines were added to the samples for analyte capturing. The immobilized target cytokine was specifically detected by biotin-conjugated antibodies and then labelled with streptavidin- Phycoerythrin (PE). The PE-labelled magnetic beads were detected and identified by LUMINEX™ 200™ analyzer. Data acquisition, processing and analysis was conducted by using XPONENT™ 3.1 software. The target cytokines were normalized to their baseline cytokine levels and presented as relative cytokines.

Figs. 1A-1C show the relative IL-6 induction from human PBMCs stimulated with various concentrations of test

compounds (Compound Nos. **2, 9** and **10)** and reference compounds **(Ref. 1-3).** Compound Nos. **2, 9** and **10** all showed low IL-6 induction activity, which are similar to **Ref. 1** and **3.** These results indicated that the compounds of the invention do not over-induce IL-6 expression, which is well-known to play a pathological effect on chronic inflammation and autoimmunity due to dysregulated continual synthesis of IL-6. To the contrary, **Ref. 2** induced high IL-6 expression when treated at a concentration over 100 nM, particular over 1000 nM, which meant that **Ref. 2** is not suitable for drug development due to a risk of over-inducing IL-6 expression. Based on the structure similarity of the Compounds **2, 9, 10** and **Ref. 2,** the low IL-6 induction activity of the compounds of the invention was unexpected.

Figs. 2A-2C show the relative IL-12p70 induction from human PBMCs stimulated with various concentrations of test compounds (Compound Nos. **2, 9** and **10)** and reference compounds **(Ref. 1-3).** Compound Nos. **2, 9** and **10** all showed high IL-12p70 induction activity, wherein the cytokine IL-12 is well known to mediate enhancement of the cytotoxic activity of NK cells and $CD8^+$ cytotoxic T cells, and anti-angiogenic activity.

Figs. 3A-3C show the relative IP-10 induction from human PBMCs stimulated with various concentrations of test compounds (Compound Nos. **2, 9** and **10)** and reference compounds **(Ref. 1-3).** Compound Nos. **2, 9** and **10** all showed high IP-10 induction activity, wherein cytokine IP-10 is well known to involve in inhibiting angiogenesis and is associated with anti-tumor activity.

Collectively, the compounds of the invention could simultaneously activate human TLR7 and TLR8, increase expression of IL-12 and IP-10 without over-inducing undesired IL-6 expression.

Table 2

| No. | Compound name | Structure |
|---|---|---|
| 1 | 1-(4-amino-2-propyl-1H-imidazo[4,5-c]quinolin-1-yl)-2-methylpropan-2-ol | 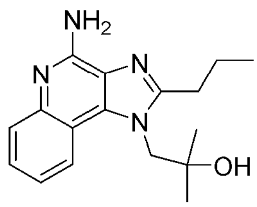 |
| 2 | 1-(4-amino-2-pentyl-1H-imidazo[4,5-c]quinolin-1-yl)-2-methylpropan-2-ol | 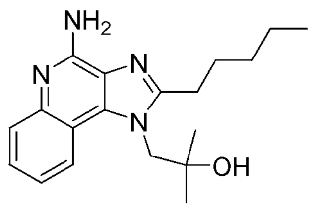 |
| 3 | 1-(4-amino-2-isobutyl-1H-imidazo[4,5-c]quinolin-1-yl)-2-methylpropan-2-ol | 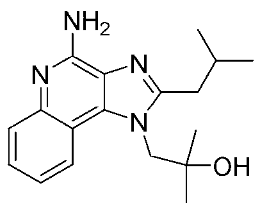 |
| 4 | 1-(4-amino-2-isopentyl-1*H*-imidazo[4,5-c]quinolin-1-yl)-2-methylpropan-2-ol | 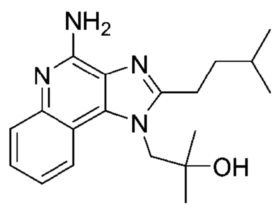 |
| 5 | 1-(4-amino-2-(4-methylpentyl)-1*H*-imidazo[4,5-c]quinolin-1-yl)-2-methylpropan-2-ol | 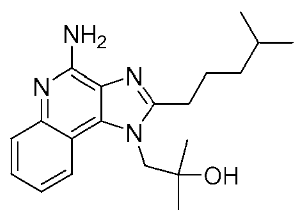 |

(continued)

| No. | Compound name | Structure |
|---|---|---|
| 6 | 1-(4-amino-2-trifluoromethyl-1*H*-imidazo[4,5-c]quinolin-1-yl)-2-methylpropan-2-ol | 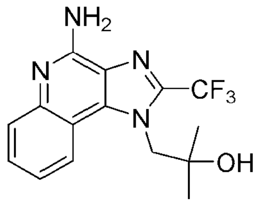 |
| 7 | 1-(4-amino-2-(2,2,2-trifluoroethyl)-1*H*-imidazo[4,5-c]quinolin-1-yl)-2-methylpropan-2-ol | 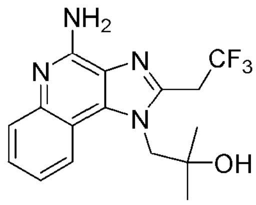 |
| 8 | 1-(4-amino-2-(3,3,3-trifluoropropyl)-1*H*-imidazo[4,5-c]quinolin-1-yl)-2-methylpropan-2-ol | 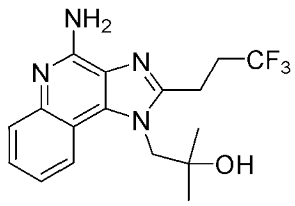 |
| 9 | 1-(4-amino-2-(4,4,4-trifluorobutyl)-1*H*-imidazo[4,5-c]quinolin-1-yl)-2-methylpropan-2-ol | 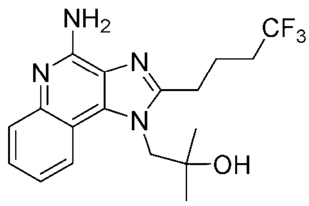 |
| 10 | 1-(4-amino-2-(5,5,5-trifluoropentyl)-1*H*-imidazo[4,5-c]quinolin-1-yl)-2-methylpropan-2-ol | 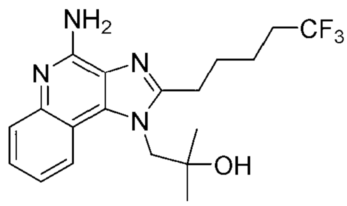 |
| 11 | 1-(4-amino-2-(6,6,6-trifluorohexyl)-1*H*-imidazo[4,5-c]quinolin-1-yl)-2-methylpropan-2-ol | 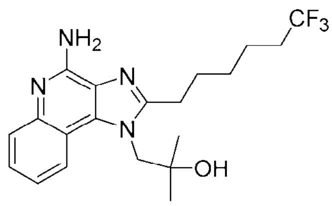 |
| 12 | 1-(4-amino-2-(3,3,3-trifluoro-2-methylpropyl)-1*H*-imidazo[4,5-c]quinolin-1-yl)-2-methylpropan-2-ol | 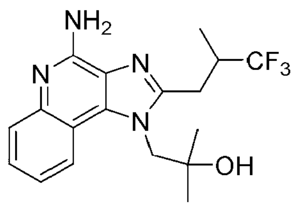 |

(continued)

| No. | Compound name | Structure |
|---|---|---|
| 13 | 1-(4-amino-2-(4,4,4-trifluoro-3-methylbutyl)-1*H*-imidazo[4,5-c]quinolin-1-yl)-2-methylpropan-2-ol | 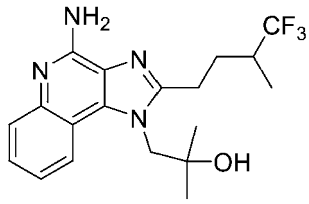 |
| 14 | 1-(4-amino-2-(5,5,5-trifluoro-4-methylpentyl)-1*H*-imidazo[4,5-c]quinolin-1-yl)-2-methylpropan-2-ol | 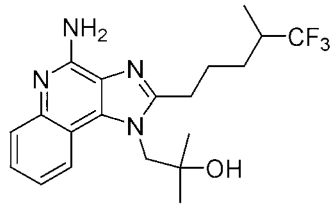 |
| 15 | 1-(4-amino-2-(2-(2,2,2-trifluoroethoxy)ethyl)-1*H*-imidazo[4,5-c]quinolin-1-yl)-2-methylpropan-2-ol | 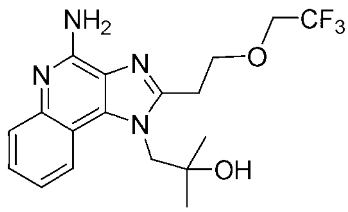 |
| 16 | 1-(4-amino-2-((3,3,3-trifluoropropoxy)methyl)-1*H*-imidazo[4,5-c]quinolin-1-yl)-2-methylpropan-2-ol | 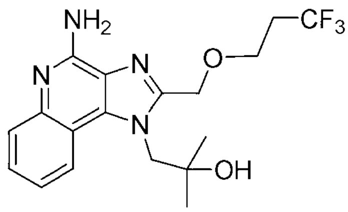 |
| 17 | 1-(4-amino-2-(((2,2,2-trifluoroethyl)thio)methyl)-1*H*-imidazo[4,5-c]quinolin-1-yl)-2-methylpropan-2-ol | 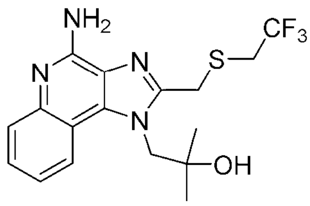 |
| 18 | 1-(4-amino-2-(((3,3,3-trifluoropropyl)thio)methyl)-1*H*-imidazo[4,5-c]quinolin-1-yl)-2-methylpropan-2-ol | 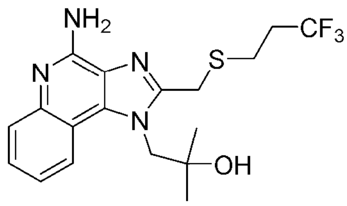 |
| 19 | 1-(4-amino-2-(((4,4,4-trifluorobutyl)thio)methyl)-1*H*-imidazo[4,5-c]quinolin-1-yl)-2-methylpropan-2-ol | 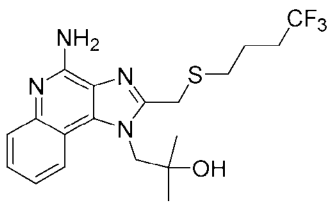 |

(continued)

| No. | Compound name | Structure |
|---|---|---|
| 20 | 1-(4-amino-2-(2-hydroxyethyl)-1*H*-imidazo[4,5-c]quinolin-1-yl)-2-methylpropan-2-ol | 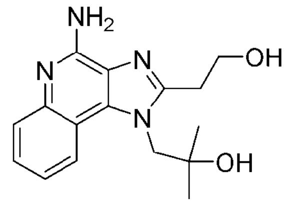 |
| 21 | 1-(4-amino-2-(3-hydroxypropyl)-1*H*-imidazo[4,5-c]quinolin-1-yl)-2-methylpropan-2-ol | 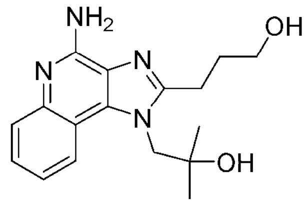 |
| 22 | 1-(4-amino-2-(4-hydroxybutyl)-1*H*-imidazo[4,5-c]quinolin-1-yl)-2-methylpropan-2-ol | 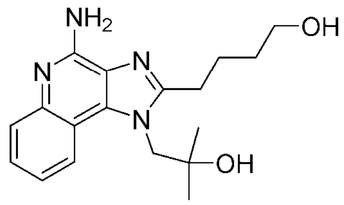 |
| 23 | 4-amino-2-butyl-1-(2-hydroxy-2-methylpropyl)-1*H*-imidazo[4,5-c]quinolin-7-ol | 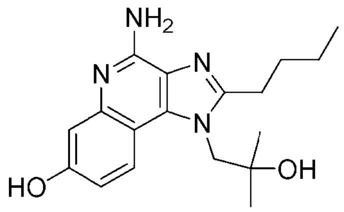 |
| 24 | 4-amino-2-(4,4,4-trifluorobutyl)-1-(2-hydroxy-2-methylpropyl)-1*H*-imidazo[4,5-c]quinolin-7-ol | 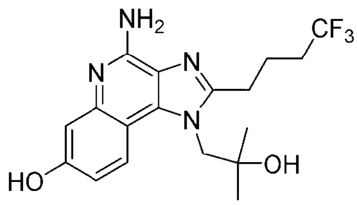 |
| 25 | 1-(4-amino-2-butyl-7-methoxy-1*H*-imidazo[4,5-c]quinolin-1-yl)-2-methylpropan-2-ol | 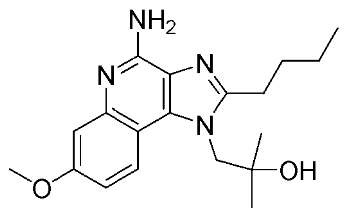 |
| 26 | 1-(4-amino-2-butyl-7-(hydroxymethyl)-1*H*-imidazo[4,5-c]quinoline-1-yl)-2-methylpropan-2-ol | 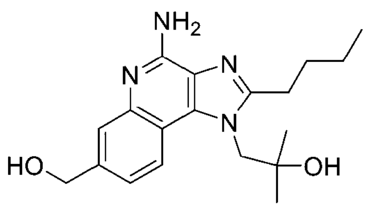 |

(continued)

| No. | Compound name | Structure |
|---|---|---|
| 27 | 1-(4-amino-2-butyl-7-fluoro-1*H*-imidazo[4,5-c]quinolin-1-yl)-2-methyl-propan-2-ol | 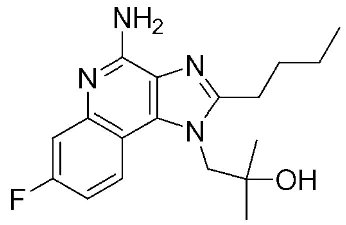 |
| 28 | 1-(4-amino-2-butyl-7-methyl-1*H*-imidazo[4,5-c]quinolin-1-yl)-2-methylpropan-2-ol | 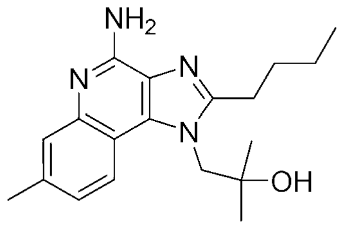 |
| 29 | 1-(4-amino-2-butyl-7-(trifluoromethyl)-1*H*-imidazo[4,5-c]quinolin-1-yl)-2-methylpropan-2-ol | 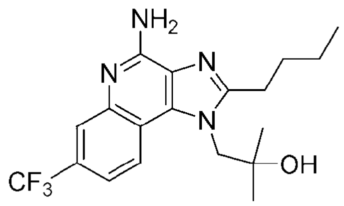 |

Toll-like receptor (TLR) ligation activates both the innate and adaptive immune systems and plays an important role in antiviral and anti-tumor immunity. The therapeutical potential of TLR agonists include use as adjuvants to vaccine, chemotherapy or standalone therapy. See Engel et. at. (2011), Expert Rev Clin Pharmacol., 4(2): 275-289.

Agonists of TLRs have been widely employed in therapeutic or prophylactic preparations useful for asthma/allergic rhinitis (AR) patients. AZD8848 (a TLR7 agonist) and VTX-1463 (a TLR8 agonist) as stand-alone therapeutics have revealed efficacy in the relief of the symptoms of AR patients. Imiquimod or R837 (a TLR7 agonist) is an FDA-approved drug used for treating skin basal cell carcinoma, actinic keratosis and external genital warts with many suggested off-label benefits in other disease, such as melanoma. Imiquimod also showed bronchodilator effects in both murine and porcine models of airway hyperresponsiveness (AHR). Imiquimod can also promote antiviral defense and protect against virus-induced airway dysfunction. It seems an interesting drug for viral-induced asthma. Resiquimod (a TLR7/8 dual agonist) is effective in suppressing acute asthma. See Aryan et al. (2014), Int Arch Allergy Immunol., 164: 46-63.

A possible link between toll-like receptor 7 (TLR7) and liver disease was suggested. The role of TLR7 signaling in liver fibrosis has been demonstrated. Imiquimod (TLR7 ligand) attenuated lipid accumulation induced by unsaturated fatty acid (UFA) and reduced lipid peroxidation products induced by UFA. *In vivo* experiments carried out with TLR7 knockout mice produced results consistent with *in vitro* experiments. The results support that TLR7 prevents progression of nonalcoholic fatty liver disease (NAFLD) via induced autophagy and released IGF-1 from liver. These findings suggest a therapeutic strategy for the treatment of NAFLD. See Kim et al. (2016), Scientific Reports, 6: 27849.

The anti-tumor activities of TLR7 agonists have been found on the tumor types including, but not limit to, squamous carcinoma, prostate cancer, bladder cancer, breast cancer, melanoma, gliomas, acute myeloid leukemia, breast cancer, T-cell lymphoma, and pancreatic cancer. The anti-tumor activities of TLR8 have been found on the tumor types including, but not limit to, gliomas, acute myeloid leukemia, breast cancer, T-cell lymphoma, lymphoma, pancreatic cancer, and colon cancer. See Chi et al (2017) Front Pharmacol., 8: 304.

Therapeutic potential/use of TLR7/8 agonists targets in different diseases, which include but not limited to treatment of viral induced lesions (caused by papilloma virus and Herpes simplex virus), primary tumors, cutaneous metastases, anti-viral (HCV), and chronic lymphocyte leukemia. See Luke et. al. (2009), Pharmacol Rev., 61(2): 177-197.

TLR7/8 agonists stimulate cells through a Toll-like receptors (TLR) 7 and 8 dependent pathway resulting in activation of immune responses that are effective against viral and tumor lesions. Resiquimod is a topical drug for viral skin lesions and skin cancer such as actinic keratosis (AK). AK, also referred as an intraepithelial SCC, is an early stage of skin cancer that has the potential to progress into an invasive squamous cell carcinoma (SCC) of the skin. Meyer et al. (2013), Expert Opin Investig Drugs, 22(1):149-159). In conclusion, the compounds of the invention are TLR7 and TLR8 dual agonists. These compounds exhibit activities in inducing IL-12 and IP-10 expression. The TLR 7/8 dual agonists are

potentially useful medications as immune response modifiers. These compounds may be used for treating a disease or a condition wherein activation of TLR7 and/or TLR8 provides a benefit in a patient, such as for use in treating a viral infection, cancer, and/or an allergic disease, or for use in activating immune responses that are effective against a viral infection, a tumor, and/or an allergic disease in a subject in need thereof.

All references cited and discussed in this specification are incorporated herein by reference in their entireties and to the same extent as if each reference was individually incorporated by reference.

## Claims

1. A compound of formula (I)

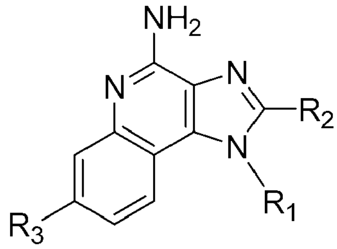

(I)

or a pharmaceutically acceptable salt thereof,
wherein
$R_1$ is hydroxy, hydroxy-$C_{1-6}$-alkyl, $C_{1-6}$-alkoxy, or $C_{1-6}$-alkoxy-$C_{1-6}$-alkyl;
$R_2$ is $C_{1-6}$-haloalkyl, $C_{1-6}$-haloalkyl-O-$C_{1-6}$-alkyl, or $C_{1-6}$-haloalkyl-S-$C_{1-6}$-alkyl; and
$R_3$ is hydrogen, hydroxy, halogen, $C_{1-6}$-alkyl, hydroxy-$C_{1-6}$-alkyl, $C_{1-6}$-haloalkyl, $C_{1-6}$-alkoxy, or $C_{1-6}$-alkoxy-$C_{1-6}$-alkyl;
wherein the $C_{1-6}$-alkyl, hydroxy-$C_{1-6}$-alkyl, $C_{1-6}$-alkoxy, $C_{1-6}$-alkoxy-$C_{1-6}$-alkyl, $C_{1-6}$-haloalkyl and $C_{1-6}$-haloalkyl-S-$C_{1-6}$-alkyl are each independently unsubstituted or substituted with at least one substituent; wherein the substituent is each independently selected from the group consisting of -$NH_2$, aryl, $C_{1-6}$-alkyl and $C_{1-6}$-alkyl-aryl.

2. The compound or the pharmaceutically acceptable salt thereof of claim 1, wherein $R_1$ is hydroxy-$C_{1-6}$-alkyl or $C_{1-6}$-alkoxy-$C_{1-6}$-alkyl.

3. The compound or the pharmaceutically acceptable salt thereof of claim 2, wherein $R_1$ is hydroxymethyl, 2-hydroxyethyl, 3-hydroxypropyl, 4-hydroxybutyl, or 2-hydroxy-2-methylpropyl.

4. The compound or the pharmaceutically acceptable salt thereof of claim 1, wherein $R_2$ is trifluoromethyl, 2,2,2-trifluoroethyl, 3,3,3-trfluoropropyl, 4,4,4-fluorobutyl, 5,5,5-trifluoropentyl, 6,6,6-trifluorohexyl, 3,3,3-trifluoro-2-methylpropyl, 4,4,4-trifluoro-3-methylbutyl, or 5,5,5-trifluoro-4-methylpentyl.

5. The compound or the pharmaceutically acceptable salt thereof of claim 1, wherein $R_2$ is-$CH_2$-$CH_2$-O-$CH_2$-$CF_3$, -$CH_2$-$CH_2$-O-$CH_2$-$CH_2$-$CF_3$, -$CH_2$-O-$CH_2$-$CF_3$, -$CH_2$-O-$CH_2$-$CH_2$-$CF_3$, or - $CH_2$-O-$CH_2$-$CH_2$-$CH_2$-$CF_3$.

6. The compound or the pharmaceutically acceptable salt thereof of claim 5, wherein $R_2$ is $C_{1-6}$-monohaloalkyl, $C_{1-6}$-dihaloalkyl, or $C_{1-6}$-trihaloalkyl.

7. The compound or the pharmaceutically acceptable salt thereof of claim 1, wherein $R_3$ is H, OH, halogen, trifluoroalkyl, $C_{1-6}$-alkyl, or hydroxy-$C_{1-6}$-alkyl.

8. The compound or the pharmaceutically acceptable salt thereof of claim 7, wherein $R_3$ is H, OH, F, Cl, Br, I, or trifluoromethyl.

9. The compound or the pharmaceutically acceptable salt thereof of claim 1, wherein $R_1$ is 2-hydroxy-2-methylpropyl, and $R_2$ is trifluoromethyl, 2,2,2-trifluoroethyl, 3,3,3-trfluoropropyl, 4,4,4-fluorobutyl, 5,5,5-trifluoropentyl, 6,6,6-trifluorohexyl, 3,3,3-trifluoro-2-methylpropyl, 4,4,4-trifluoro-3-methylbutyl, 5,5,5-trifluoro-4-methylpentyl, -$CH_2$-$CH_2$-O-$CH_2$-$CF_3$, -$CH_2$-$CH_2$-O-$CH_2$-$CH_2$-$CF_3$, -$CH_2$-O-$CH_2$-$CF_3$, -$CH_2$-O-$CH_2$-$CH_2$-$CF_3$, or -$CH_2$-O-$CH_2$-$CH_2$-$CH_2$-$CF_3$,

$-CH_2-CH_2-S-CH_2-CF_3$, $-CH_2-CH_2-S-CH_2-CH_2-CF_3$, $-CH_2-S-CH_2-CF_3$, $-CH_2-S-CH_2-CH_2-CF_3$, or $-CH_2-S-CH_2-CH_2-CH_2-CF_3$.

**10.** The compound or the pharmaceutically acceptable salt thereof of claim 1, wherein $R_1$ is 2-hydroxy-2-methylpropyl, and $R_2$ is trifluorobutyl, trifluoropentyl or trifluoro-(2-methylpropyl).

**11.** The compound or the pharmaceutically acceptable salt thereof of claim 1, wherein $R_2$ is trifluoromethyl, trifluoroethyl, trifluoropropyl, trifluorohexyl, trifluoro-3-methylbutyl, trifluoro-4-methylpentyl, ((2,2,2-trifluoroethyl)thio)methyl, ((3,3,3-trifluoropropyl)thio)methyl, ((4,4,4-trifluorobutyl)thio)methyl, or trifluorobutyl.

**12.** The compound or the pharmaceutically acceptable salt thereof of claim 1, said compound being selected from the group consisting of

1-(4-amino-2-trifluoromethyl-1*H*-imidazo[4,5-c]quinolin-1-yl)-2-methylpropan-2-ol;
1-(4-amino-2-(2,2,2-trifluoroethyl)-1*H*-imidazo[4,5-c]quinolin-1-yl)-2-methylpropan-2-ol;
1-(4-amino-2-(3,3,3-trifluoropropyl)-1*H*-imidazo[4,5-c]quinolin-1-yl)-2-methylpropan-2-ol;
1-(4-amino-2-(4,4,4-trifluorobutyl)-1*H*-imidazo[4,5-c]quinolin-1-yl)-2-methylpropan-2-ol;
1-(4-amino-2-(5,5,5-trifluoropentyl)-1*H*-imidazo[4,5-c]quinolin-1-yl)-2-methylpropan-2-ol;
1-(4-amino-2-(6,6,6-trifluorohexyl)-1*H*-imidazo[4,5-c]quinolin-1-yl)-2-methylpropan-2-ol;
1-(4-amino-2-(3,3,3-trifluoro-2-methylpropyl)-1*H*-imidazo[4,5-c]quinolin-1-yl)-2-methylpropan-2-ol;
1-(4-amino-2-(4,4,4-trifluoro-3-methylbutyl)-1*H*-imidazo[4,5-c]quinolin-1-yl)-2-methylpropan-2-ol;
1-(4-amino-2-(5,5,5-trifluoro-4-methylpentyl)-1*H*-imidazo[4,5-c]quinolin-1-yl)-2-methylpropan-2-ol;
1-(4-amino-2-(2-(2,2,2-trifluoroethoxy)ethyl)-1*H*-imidazo[4,5-c]quinolin-1-yl)-2-methylpropan-2-ol;
1-(4-amino-2-((3,3,3-trifluoropropoxy)methyl)-1*H*-imidazo[4,5-c]quinolin-1-yl)-2-methylpropan-2-ol;
1-(4-amino-2-(((2,2,2-trifluoroethyl)thio)methyl)-1*H*-imidazo[4,5-c]quinolin-1-yl)-2-methylpropan-2-ol;
1-(4-amino-2-(((3,3,3-trifluoropropyl)thio)methyl)-1*H*-imidazo[4,5-c]quinolin-1-yl)-2-methylpropan-2-ol;
1-(4-amino-2-(((4,4,4-trifluorobutyl)thio)methyl)-1*H*-imidazo[4,5-c]quinolin-1-yl)-2-methylpropan-2-ol; and
4-amino-2-(4,4,4-trifluorobutyl)-1-(2-hydroxy-2-methylpropyl)-1*H*-imidazo[4,5-c]quinolin-7-ol.

**13.** A compound or a pharmaceutically acceptable salt thereof as claimed in any one of claims 1-12 for use in treating a viral infection, cancer, an allergic disease, or a liver disease in a subject in need thereof.

**14.** The compound or the pharmaceutically acceptable salt thereof of claim 13, wherein the cancer is selected from the group consisting of esophageal, stomach, colon, rectal, pancreatic, lung, breast, cervix uteri, corpus uteri, ovary, bladder, head and neck, endometrial, osteosarcoma, prostate, and neuroblastoma.

**15.** A compound or a pharmaceutically acceptable salt thereof as claimed in any one of claims 1-12 for use in treating a disease or a condition wherein activation of TLR7 and/or TLR8 provides a benefit in a subject in need thereof.

Fig. 1A

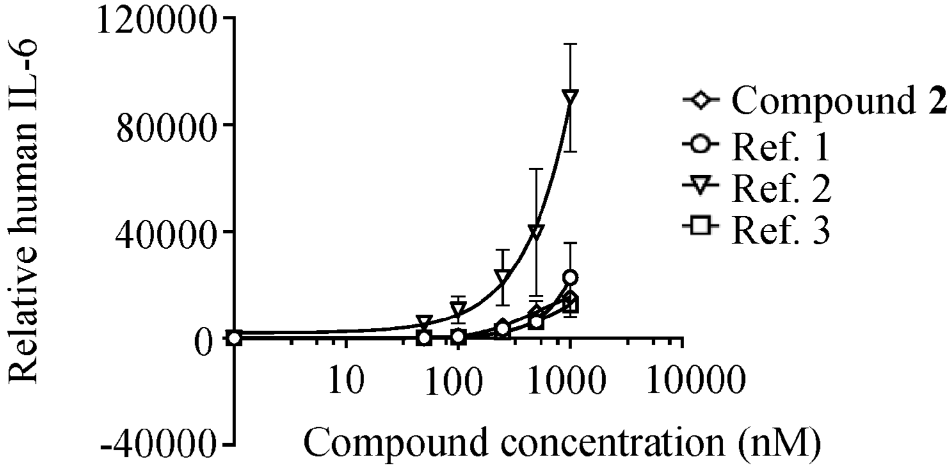


Fig. 1B

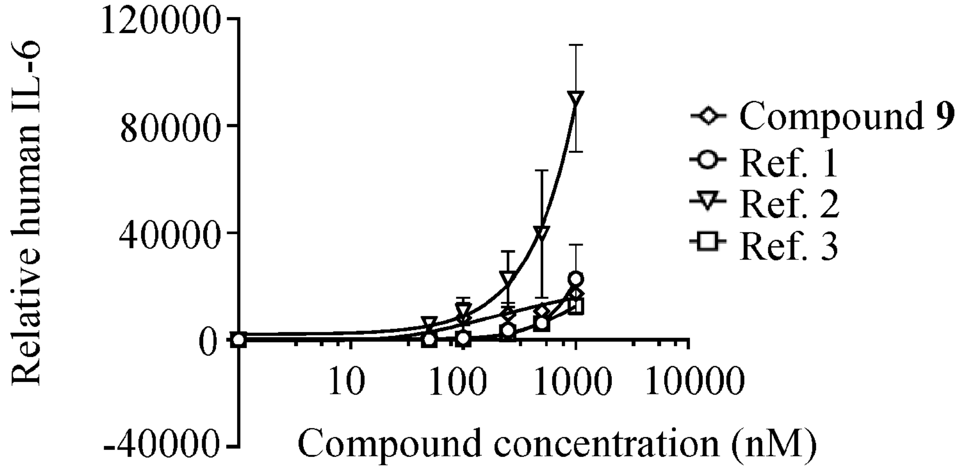


Fig. 1C

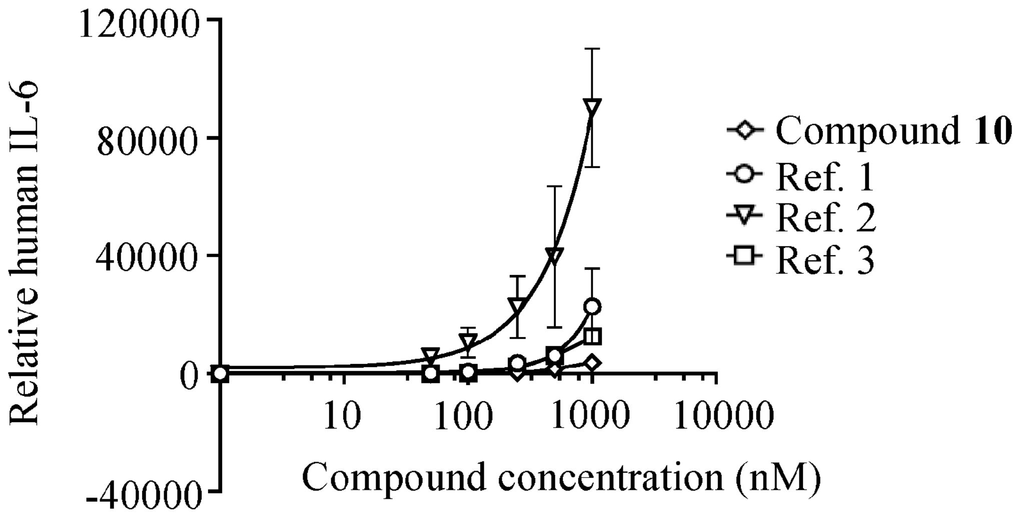

Fig. 2A

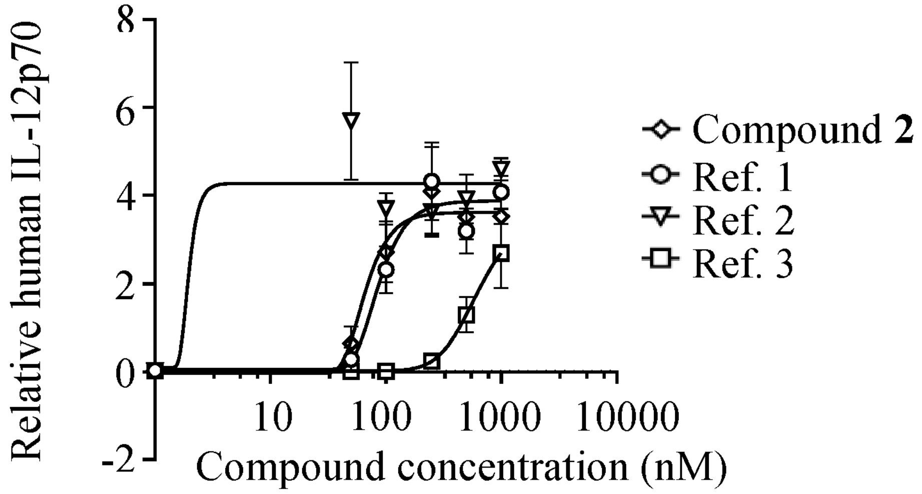


Fig. 2B

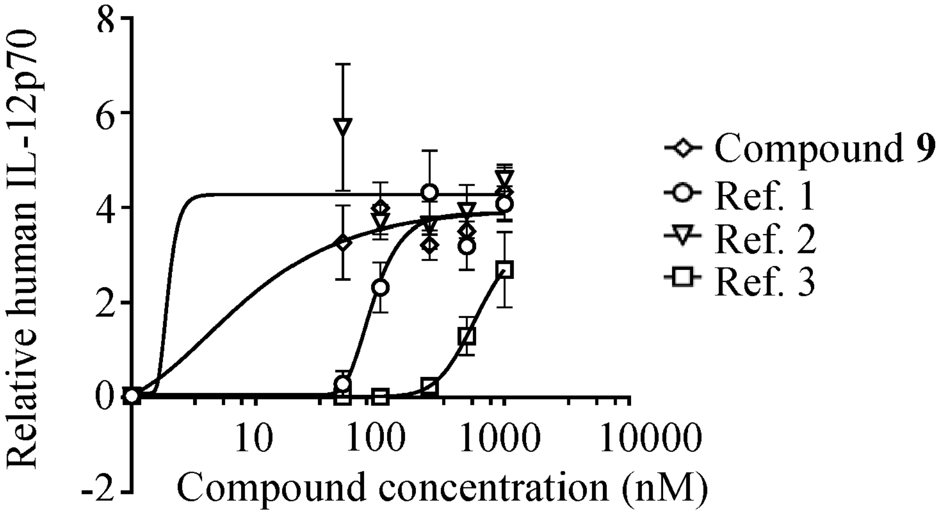


Fig. 2C

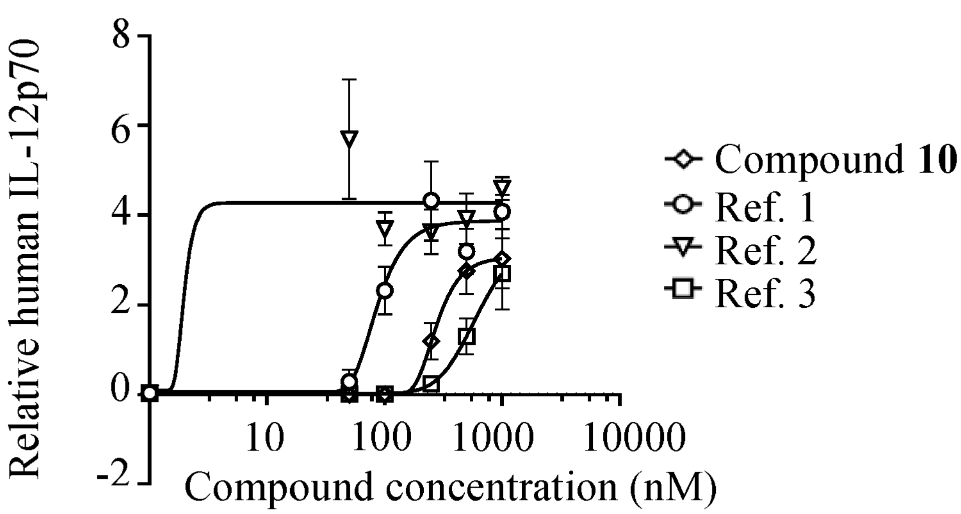

Fig. 3A

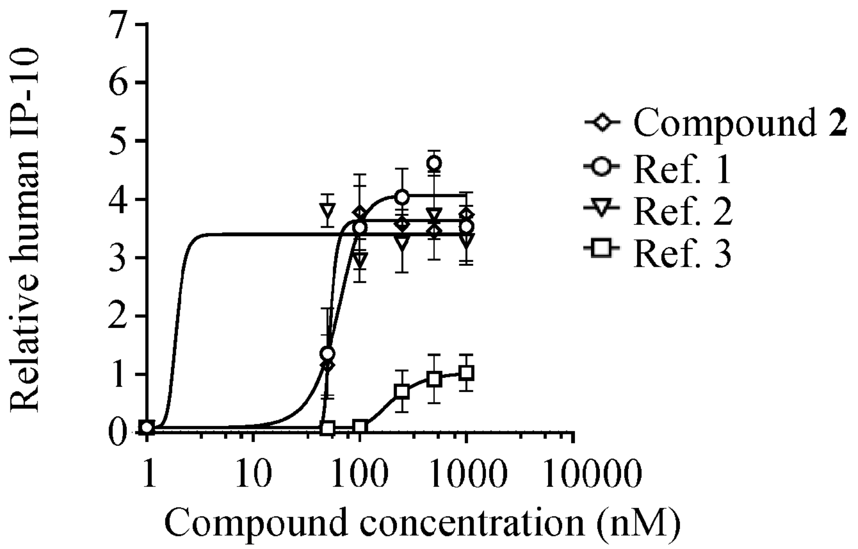


Fig. 3B

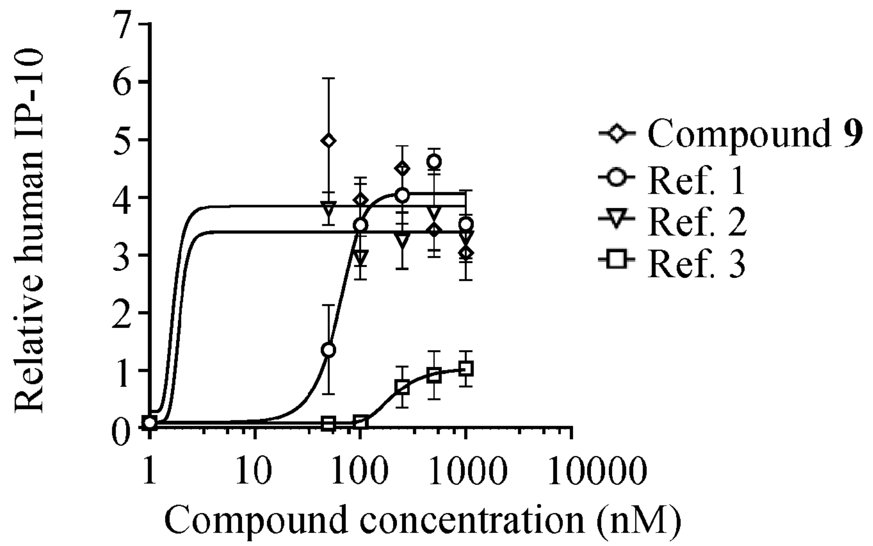


Fig. 3C

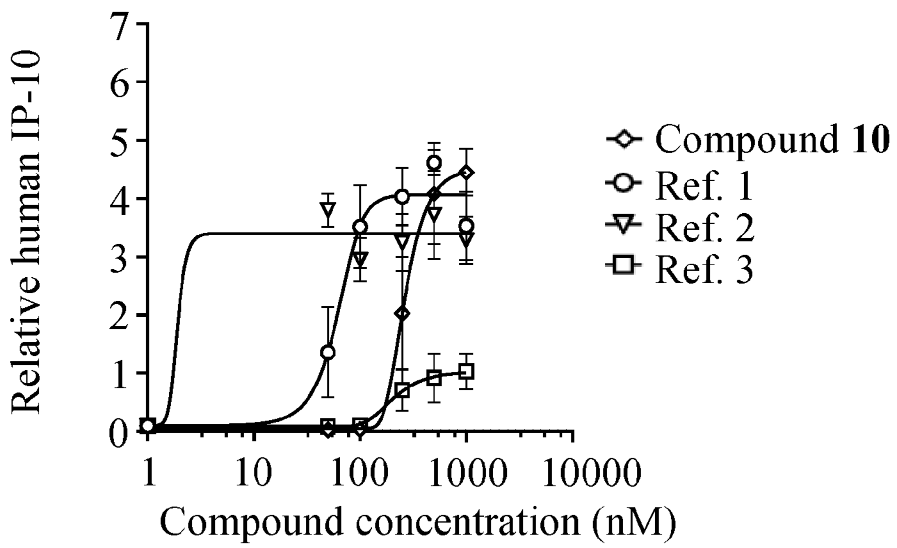

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8728486 B
- US 9334268 B
- US 9884866 B
- US 20190062329 A


### Non-patent literature cited in the description


- **ENGEL**. *Expert Rev Clin Pharmacol.*, 2011, vol. 4 (2), 275-289
- **ARYAN et al.** *Int Arch Allergy Immunol.*, 2014, vol. 164, 46-63
- **KIM et al.** *Scientific Reports*, 2016, vol. 6, 27849
- **CHI et al.** *Front Pharmacol.*, 2017, vol. 8, 304
- **LUKE**. *Pharmacol Rev.*, 2009, vol. 61 (2), 177-197
- **MEYER et al.** *Expert Opin Investig Drugs*, 2013, vol. 22 (1), 149-159